Europäisches Patentamt

European Patent Office

Office européen des brevets

(19)

(11) Numéro de publication : **0 425 373 B1**

(12) **FASCICULE DE BREVET EUROPEEN**

(45) Date de publication du fascicule du brevet :
03.02.93 Bulletin 93/05

(51) Int. Cl.$^5$ : **A61K 9/16, A61K 47/34**

(21) Numéro de dépôt : **90402994.9**

(22) Date de dépôt : **24.10.90**

(54) **Compositions cosmétique et pharmaceutique contenant des sphères de poly-bêta-alanine réticulée, impregnées d'une substance lipophile.**

(30) Priorité : **24.10.89 FR 8913938**

(43) Date de publication de la demande :
**02.05.91 Bulletin 91/18**

(45) Mention de la délivrance du brevet :
**03.02.93 Bulletin 93/05**

(84) Etats contractants désignés :
**AT BE CH DE DK ES FR GB GR IT LI NL SE**

(56) Documents cités :
**WO-A-87/02684**
**FR-A- 2 530 250**

(73) Titulaire : **L'OREAL**
**14, Rue Royale**
**F-75008 Paris (FR)**

(72) Inventeur : **Lapoirière, Vandenbossche, Claudine**
**3 rue de Lyser**
**F-94170 Le Perreux (FR)**
Inventeur : **Mahieu, Claude**
**90, avenue de Villiers**
**F-75017 Paris (FR)**
Inventeur : **Papantoniou, Christos**
**17, rue des Basserons**
**F-95160 Montmorency (FR)**

(74) Mandataire : **Stalla-Bourdillon, Bernard et al**
**CABINET NONY & CIE 29, rue Cambacérès**
**F-75008 Paris (FR)**

EP 0 425 373 B1

Jouve, 18, rue Saint-Denis, 75001 PARIS

## Description

La présente invention a pour objet une composition cosmétique ou pharmaceutique se présentant sous la forme d'un gel ou d'une poudre, contenant des sphères de poly β-alanine réticulée imprégnées d'une substance lipophile.

Dans FR-A- 2.530.250 on a décrit la préparation de la poly β-alanine réticulée insoluble dans l'eau, celle ci étant obtenue par réticulation de la poly β-alanine soluble dans l'eau, à l'aide de divers agents réticulants en particulier de dialdéhydes tels que le glutaraldéhyde.

La poly β-alanine réticulée présentant la particularité de gonfler à l'eau et d'en retenir une quantité importante, celle-ci a trouvé une utilisation dans le domaine thérapeutique par application sur des plaies suintantes infectées ou non, ceci de façon à favoriser l'absorption et par conséquent le processus naturel de cicatrisation.

Par ailleurs, il a également été proposé d'utiliser la poly β-alamine réticulée comme support pour certaines substances actives hydrosolubles.

Selon ce mode de réalisation, les sphères de poly β-alanine réticulée peuvent être imprégnées par certains produits cicatrisants en solution aqueuse ce qui provoque leur gonflement. Le produit obtenu, qui se présente alors sous forme d'une pâte, peut être appliqué sur la plaie et facilite ainsi les échanges entre les liquides de la plaie et la solution contenue dans la pâte.

Il est également possible après l'imprégnation des sphères de poly β-alanine réticulée à l'aide de la substance active hydrosoluble, de procéder à un séchage, ce qui permet d'emprisonner la substance active dans les sphères. Lorsque la substance active est un agent cicatrisant, cette forme de réalisation, permet donc, tout en assainissant les plaies d'en assurer une meilleure cicatrisation.

Il a également été proposé d'utiliser les sphères de poly β-alanine réticulée comme excipient cosmétique ou pharmaceutique et dans ce cas les sphères améliorent la consistance et favorisent également certains traitements par voie topique.

Dans FR-A- 2.624.866 on a décrit un nouveau procédé de préparation de la poly β-alanine réticulée sous forme de microsphères ayant une très faible dispersité de taille, ce qui permet d'éviter l'étape longue et laborieuse du tamisage du procédé décrit dans FR-A-2.530.250.

Les sphères de poly β-alanine réticulée ne présentant aucune affinité pour les huiles, leur imprégnation reste limitée à des substances hydrosolubles ou solubles dans des alcools polyhydroxylés ou encore solubles dans des mélanges d'eau avec des solvants miscibles à l'eau comme l'acétone, l'éthanol ou le tétrahydrofuranne. Toutefois lorsque l'on utilise ces derniers solvants, ceux-ci doivent être éliminés avant la formulation dans des compositions cosmétiques ou pharmaceutiques ce qui complique le processus d'imprégnation des sphères.

Après d'importantes recherches, et de façon tout à fait inattendue, on a constaté que les sphères de poly β-alanine réticulée pouvaient en fait être imprégnées d'une substance lipophile telle qu'une huile, d'une huile essentielle ou d'un produit cireux en soumettant au préalable les sphères de poly β-alanine réticulée à un traitement particulier.

En effet, de façon surprenante, on a observé qu'en imprégnant au préalable les sphères de poly β-alanine à l'aide d'eau, d'un alcool polyhydroxylé ou d'un de leurs mélanges, ceci permettait de retenir sur la surface des sphères des quantités relativement importantes d'une substance lipophile lorqu'elles étaient mises en contact avec cette dernière.

La présente invention a donc pour objet une composition cosmétique, sous forme d'un gel ou d'une poudre, contenant en dispersion des sphères de poly β-alanine réticulée imprégnées d'une substance lipophile.

Selon l'invention on utilise de préférence des microsphères de poly β-alanine réticulée obtenues selon le procédé FR-A-2.624.866, avec un excellent rendement et présentant par ailleurs une très faible dispersité de taille.

Selon l'invention on peut également utiliser des microsphères de poly β-alanine réticulée, ayant une faible dispersité de taille, obtenues par polymérisation de l'acrylamide avec un composé polyfonctionnel copolymérisable avec l'acrylamide en tant qu'agent réticulant, dans un solvant organique ou un mélange de tels solvants en présence d'un initiateur anionique de polymérisation et d'un agent de suspension.

Selon ce procédé les composés polyfonctionnels sont de préférence choisis parmi les suivants : 1,2-dihydroxyéthylène bis-acrylamide, N-N'-(bis-acrylyl) cystéamine, diméthylène bis-acrylamide, hexaméthylène bis-acrylamide, octaméthylène bis-acrylamide, dodécaméthylène bis-acrylamide, m-xylylène bis-acrylamide, butylidène bis-acrylamide, benzylidène bis-acrylamide, acide diacrylamido acétique, xylylidène tétra-acrylamide, N,N'-bis-(acrylyl) pipérazine et p-benzylidène camphre N,N'-acrylyl diaminométhane.

Le solvant organique est de préférence le tertiobutanol ou le toluène et les mélanges tertio-butanol/toluène dans des rapports compris entre 1:24 et 10:1 et de préférence entre 1:6 et 6:1, et l'initiateur anionique de polymérisation est le tertio-butylate de sodium ou de potassium utilisé en une proportion de 0,1 à 2 moles % par rapport à l'acrylamide.

Selon ce procédé l'agent de suspension est de préférence le copolymère octadécène-anhydride maléïque.

Pour l'imprégnation à l'aide des substances lipophiles, les sphères sont soumises à une étape de gonflement à l'aide d'eau, d'un alcool polyhydroxylé, ou d'un de leurs mélanges, mais de préférence à l'aide d'eau ou d'un mélange d'eau et d'un alcool polyhydroxylé tel que par exemple le glycérol ou le propanediol-1,2, dans des proportions comprises entre une (1) et dix (10) fois la masse des sphères.

La substance lipophile est alors mise en contact avec les sphères de préférence sous agitation vive, et la quantité de substance lipophile que l'on peut fixer sur les sphères dépend alors non seulement de sa nature mais également de la quantité de solvant aqueux absorbée par les sphères.

De façon générale, l'imprégnation par la substance lipophile est réalisée à température ambiante ou éventuellement à température plus élevée mais il convient que celle ci ne dépasse pas 100°C lorsque les sphères ont été préalablement imprégnées d'eau. Par contre lorsque les sphères ont été imprégnées à l'aide d'un alcool polyhydroxylé ou d'un mélange d'eau et d'un alcool polyhydroxylé, il est alors possible d'utiliser des températures supérieures à 100°C sous réserve toutefois que les propriétés physicomécaniques des microsphères ne soient pas modifiées.

Il va de soi que la substance lipophile peut servir également de véhicule à une ou plusieurs substances actives, cosmétiques ou pharmaceutiques, liposolubles.

Parmi les substances liposolubles on pourra citer notamment :

Comme antifongiques, l'éconazol et le miconazole,

comme antibactériens, le chlorquinol, l'hexachlorophène, le 2,4,4′-trichloro-2′-hydroxybiphényléther et l'acide usnique.

comme antipelliculaire, le goudron de houille,

comme antiséborrhéique, la thioxolone,

comme antiacnéiques, l'acide rétinoïque et ses dérivés,

comme kératolytique, l'acide salicylique,

comme antipsoriatique, l'anthraline

Il est également possible selon un mode de réalisation préféré de l'invention, de réaliser le gonflement des sphères de poly β-alanine réticulée à l'aide d'eau ou d'un mélange d'eau et d'un alcool polyhydroxylé contenant en solution une substance active non liposoluble.

Lorsque la substance active non liposoluble présente une solubilité insuffisante dans les solvants ci-dessus énumérés on pourra, malgré les inconvénients précédemment mentionnés, rajouter des solvants miscibles à l'eau tels que l'éthanol, l'acétone ou le tétrahydrofuranne au cours de la phase de gonflement. Ces solvants, notamment l'acétone et le tétrahydrofuranne, seront éliminés par évaporation avant la phase d'imprégnation avec la substance lipophile, à titre d'exemple on citera notamment le peroxyde de benzoyle et le minoxidil.

Selon cette forme de réalisation, on peut donc obtenir des sphères imprégnées à la fois d'une substance active non liposoluble et d'une substance active liposoluble.

Parmi les substances lipophiles qui peuvent imprégner sur les sphères de poly β-alanine réticulée, on peut notamment mentionner parmi les huiles et les beurres, l'huile de paraffine, l'huile d'arachide, une huile de silicone, l'huile de palme, le beurre de karité, etc...

Parmi les huiles essentielles qui peuvent être associées aux sphères de poly β-alanine réticulée on peut notamment mentionner les extraits de rocou, les extraits de parfum tels que ceux connus sous les dénominations commerciales de "Loulou", de la Société CACHAREL.

Parmi les substances actives qui peuvent être associées aux sphères de poly β-alanine réticulée, avec ou sans utilisation d'une huile comme solvant, on peut notamment mentionner l'acétate d'α-tocophérol, le nicotinate d'α-tocophérol, le β-carotène, le N,N-diéthyltoluamide, des filtres solaires tels que le "Parsol MCX" vendu par la Société GIVAUDAN, "l'Uvinul-40" vendu par la Société BASF, le ricinoléate de zinc, etc...

La présente invention a également pour objet le procédé de préparation des sphères de poly β-alanine réticulée imprégnées d'une substance lipophile.

Ce procédé, comme indiqué ci-dessus, consiste en un premier temps à faire gonfler les sphères de poly β-alanine réticulée, ayant de préférence une faible dispersité de taille, avec de l'eau, un alcool polyhydroxylé ou un de leurs mélanges, ou encore avec de l'eau et un solvant choisi parmi l'éthanol, l'acétone ou le tétrahydrofuranne, et à mettre en contact les sphères ainsi gonflées en présence de la substance lipophile soit seule soit associée à un actif liposoluble.

Les sphères de poly β-alanine réticulée ainsi imprégnées se présentent généralement sous la forme d'un produit ayant une consistance poudreuse ou semi-pâteuse que l'on disperse ensuite dans le véhicule cosmétique ou pharmaceutique, celui-ci étant de préférence un gel ou une poudre.

Il importe en effet, que le véhicule soit exempt d'une phase huileuse miscible à la substance lipophile ceci de façon à éviter tout échange entre les sphères imprégnées d'une substance lipophile et la phase huileuse.

Dans les compositions sous forme de gel selon l'invention, la proportion en sphères de poly β-alanine réticulée imprégnées d'une substance lipophile peut varier dans de larges limites mais est généralement comprise entre 0,1 et 60 % en poids et de préférence de 4 à 20 % par rapport au poids total du gel.

L'agent gélifiant est généralement présent à une concentration comprise entre 0,1 et 5 % par rapport au poids total du gel.

Parmi les agents gélifiants particulièrement préférés on peut notamment mentionner :
- les polymères carboxyvinyliques tels que le "CARBOPOL® 940", "CARBOPOL® 934", "CARBOPOL® 934 DM",
- les polymères cellulosiques tels que les "KLUCEL HF", le "NATROSOL", la cellulose gum, commercialisées par la Société HERCULES.
- les gommes de xanthane.

Dans les compositions sous forme de poudre selon l'invention, la proportion en sphères de poly β-alanine réticulée imprégnées d'une substance lipophile peut varier dans de larges limites mais est généralement comprise entre 0,1 et 60 % en poids mais de préférence entre 0,5 et 40 % par rapport au poids total de la composition.

Les poudres selon l'invention contiennent en plus des sphères de poly β-alanine réticulée imprégnées d'une substance lipophile, au moins une charge minérale ou organique telle que du talc, de l'amidon etc... en une proportion d'environ 5 à 95 % en poids, et éventuellement au moins un agent liant comme le carbonate de magnésium. Ce dernier peut-être aussi un corps gras à condition toutefois qu'il ne soit pas un solvant de la substance lipophile qui imprégné les sphères.

Selon une forme particulière de réalisation, les poudres peuvent être formulées sous forme d'une bombe aérosol, notamment d'un déodorant.

On va maintenant donner à titre d'illustration plusieurs exemples de préparation des sphères de poly β-alanine réticulée imprégnées d'une substance lipophile ainsi que plusieurs exemples des gels et des poudres selon l'invention contenant lesdites sphères.

## EXEMPLES DE PREPARATIONS DE SPHERES DE POLY β-ALANINE RETICULEE IMPREGNEE D'UNE SUBSTANCE LIPOPHILE

Dans les exemples suivants la poly β-alanine réticulée utilisée a été obtenue soit selon le procédé du FR-A-2.624.866 soit selon les exemples A et B suivants :

## EXEMPLE A

Dans un réacteur de 2 litres, muni d'une agitation mécanique à 500t/min, d'un réfrigérant, d'une arrivée d'azote et d'un moyen de régulation de la température, on introduit 300 g de toluène, 147 g de tertiobutanol et 0,5 g de copolymère octadécène-anhydride maléïque (vendu sous la dénomination de "PA-18" par la Société GULF). Après chauffage de ce mélange à 70°C on ajoute 45 g d'acrylamide et 5 g de butylidène bis-acrylamide dans 50 g de toluène. On porte alors la température à 80°C pendant 15 min. environ. On ajoute ensuite en 10 min. une solution de 1,12 g de tertiobutylate de potassium dans 20 g de tertiobutanol, celle-ci ayant été obtenue en portant le mélange au reflux pendant 2 heures.

Après agitation pendant 6 heures à 80°C, on laisse revenir à température ambiante puis on filtre les microsphères de poly β-alanine réticulée. Après lavage à l'éthanol et à l'eau, les microsphères de poly β-alanine réticulée sont ensuite séchées par lyophilisation (rendement 40 %). Les microsphères de poly β-alanine réticulée se présentent sous forme d'une poudre blanche, dont le diamètre moyen des microsphères est en moyenne de 6 μm déterminé par la technique d'analyse d'images sur un appareil "QUANTIMET 900" de la Société CAMBRIDGE INSTRUMENTS CO.

## EXEMPLE B

Dans un réacteur de 2 litres, muni d'une agitation mécanique a 500t/min., d'un réfrigérant, d'une arrivée d'azote et d'un moyen de régulation de la température, on introduit 300 g de toluène, 147 g de tertiobutanol et 0,5 g de copolymère octadécène-anhydride maléïque (vendu sous la dénomination de "PA-18" par la Société GULF). Après chauffage de ce mélange à 70°C on ajoute 48,75 g d'acrylamide et 1,25 g de diméthylène bis-acrylamide dans 50 g de toluène. On porte alors la température à 80°C pendant 15 min. environ. On ajoute ensuite en 10 min. une solution de 1,12 g de tertiobutylate de potassium dans 20 g de tertiobutanol, celle-ci ayant été obtenue en portant le mélange au reflux pendant 2 heures.

Après agitation pendant 5 heures à 80°C, on laisse revenir à température ambiante puis on filtre les microsphères de poly β-alanine réticulée. Après lavage à l'éthanol et à l'eau, les microsphères de poly β-alanine réticulée sont ensuite séchées par lyophilisation (poids obtenu : 40,5 g). Les microsphères de poly β-alanine réticulée se présentent sous forme d'une poudre blanche, dont le diamètre moyen des microsphères est de 7 μm déterminé par la technique d'analyse d'images sur un appareil "QUANTIMET 900" de la Société CAMBRIDGE INSTRUMENTS CO.

EXEMPLE 1

A 43,2 g de sphères de poly β-alanine réticulée à l'état sec, obtenues selon FR-A- 2.624.866, on ajoute une quantité d'eau suffisante de telle sorte que l'on obtienne 200 g de sphères hydratées, puis l'on introduit 43,2 g d'huile de paraffine fluide et l'on agite jusqu'à l'obtention d'un mélange homogène.
On obtient ainsi un produit semi-poudreux facilement dispersible dans l'eau.

EXEMPLE 2

A 10 g de sphères de poly β-alanine réticulée, obtenues selon l'exemple A ci-dessus, on ajoute une quantité d'eau suffisante de telle sorte que l'on obtienne 44 g de sphères hydratées et on ajoute ensuite 10 g d'une huile de silicone volatile vendue sous la dénomination de "ABIL-K4" par la société Goldschmidt. On agite l'ensemble à l'aide d'un agitateur Rayneri muni d'une défloculeuse jusqu'à l'obtention d'un mélange homogène.
On obtient ainsi un produit semi-poudreux facilement dispersible dans l'eau.

EXEMPLE 3

A 10 g de sphères de poly β-alanine réticulée, obtenues selon l'exemple B ci-dessus, on ajoute une quantité d'eau suffisante telle que l'on obtienne 44 g de sphères hydratées et l'on ajoute ensuite 10 g d'une huile de silicone vendue sous la dénomination de "SILICON 7205" par la Société Union Carbide.
Après mélange à l'aide d'un agitateur Rayneri muni d'une défloculeuse on obtient un mélange homogène ayant un aspect semi-poudreux.

EXEMPLE 4

Dans un réacteur on introduit 15 g de poly β-alanine réticulée à l'état sec, obtenues selon l'exemple A ci-dessus, puis une quantité d'eau suffisante de telle sorte que l'on obtienne 66,3 g de sphères hydratées. Après agitation sous azote et chauffage jusqu'à 80°C, on ajoute alors 30 g d'acétate d'$\alpha$-tocophérol préalablement chauffé sous azote à 70°C. On agite ensuite jusqu'à l'obtention d'un mélange homogène et on laisse refroidir. On obtient ainsi un mélange à l'état pâteux facilement dispersible dans l'eau.

EXEMPLE 5

Dans un réacteur on introduit 15 g de poly β-alanine réticulée à l'état sec, obtenues selon FR-A-2.624.866, et une quantité d'eau suffisante pour obtenir 66,3 g de sphères hydratées. On agite sous azote et chauffe jusqu'à 80 °C. On ajoute alors 15 g d'acétate d'$\alpha$-tocophérol préalablement chauffé sous azote à 70°C. On agite alors jusqu'à l'obtention d'un mélange homogène et on laisse refroidir.
On obtient alors un mélange pâteux facilement dispersible dans l'eau.

EXEMPLE 6

Dans un réacteur on introduit 5 g de sphères de poly β-alanine réticulée à l'état sec, obtenues selon l'exemple B ci-dessus, et la quantité d'eau suffisante de sorte que l'on obtienne 21,1 g de sphères hydratées. Après avoir agité sous azote et chauffé jusqu'à une température de 80 °C, on ajoute 5 g de nicotinate d'$\alpha$-tocophérol préalablement fondu et maintenu à 70°C sous azote. On poursuit l'agitation jusqu'à l'obtention d'un mélange homogène et on laisse refroidir. On obtient ainsi un produit pâteux facilement dispersible dans l'eau.

EXEMPLE 7

A 10 g de sphères de poly β-alanine réticulée à l'état sec, obtenues selon FR-A-2.624.866, on ajoute la quantité d'eau suffisante de façon à obtenir 44,2 g de sphères hydratées. On ajoute alors sous agitation 10 g d'une suspension à 30 % de β-carotène dans de l'huile d'arachide. On mélange alors à l'agitateur Rayneri muni d'une défloculeuse et on obtient un produit semi-pâteux facilement dispersible dans l'eau.

## EXEMPLE 8

A 10 g de sphères de poly β-alanine réticulée, obtenues selon FR-A-2.624.866, on ajoute la quantité d'eau suffisante de façon à obtenir 47,2 g de sphères hydratées. On ajoute alors 10 g d'huile de Rocou vendue sous la dénomination de "R400S" par la Société Boll.

Après homogénéisation à l'aide d'un agitateur Rayneri muni d'une défloculeuse, on obtient un produit pâteux facilement dispersible dans l'eau.

## EXEMPLE 9

A 3 g de sphères de poly β-alanine réticulée à l'état sec, obtenues selon l'exemple A ci-dessus, on ajoute la quantité d'eau suffisante de façon à obtenir 13,5 g de sphères hydratées. On ajoute 3 g de N,N-diéthyl-m-toluamide, puis on procède à l'homogénéisation par agitation à l'aide d'un agitateur Rayneri muni d'une défloculeuse.

On obtient ainsi un produit semi-poudreux facilement dispersible dans l'eau.

## EXEMPLE 10

A 3 g de sphères de poly β-alanine réticulée, obtenues selon l'exemple B ci-dessus, on ajoute la quantité d'eau suffisante de façon à obtenir 13,5 g de sphères hydratées. On ajoute alors 3 g d'un agent anti-solaire vendu sous la dénomination de "Parsol MCX" (p-méthoxycinnamate d'éthyl 2-hexyle) vendu par la Société GIVAUDAN.

Après mélange à l'aide d'une spatule on obtient un produit pâteux d'aspect homogène.

## EXEMPLE 11

A 22,6 g de sphères de poly β-alanine réticulée à l'état sec, obtenues selon FR-A-2.624.866, on ajoute la quantité d'eau suffisante de façon à obtenir 100 g de sphères hydratées. On ajoute 45,2 g d'un agent anti-solaire de dénomination "Escalol" (amino-4 benzoate d'éthyl-2 hexyle) vendu par la Société GIVAUDAN.

Après mélange à l'agitateur Rayneri muni d'une défloculeuse on obtient un produit ayant un aspect semi-pâteux facilement dispersible dans l'eau.

## EXEMPLE 12

A 5 g de sphères de poly β-alanine réticulée à l'état sec, obtenues selon l'exemple A ci-dessus, on ajoute la quantité d'eau suffisante de façon à obtenir 22,1 g de microsphères hydratées. Après chauffage à 70°C sous agitation, on ajoute 5 g de nicotinate d'α-tocophérol fondu à 70°C.

Après agitation à l'aide d'un agitateur Rayneri muni d'une défloculeuse, on obtient un produit homogène à l'aspect semi-pâteux.

## EXEMPLE 13

A 20 g de sphères de poly β-alanine réticulée à l'état sec, obtenues selon FR-A-2.624.866, on ajoute la quantité d'eau suffisante de façon à obtenir 20,6 g de sphères hydratées.

On ajoute 20 g d'un mélange absorbeur d'odeur vendu par la Société GRILLO-WERKE sous le nom de "GRILLOCIN HY77".

Le produit obtenu, après homogénéisation au Rayneri est un produit semi poudreux.

## EXEMPLE 14

A 7,5 g de sphères de poly β-alanine réticulée, obtenues selon l'exemple A ci-dessus, on ajoute la quantité d'eau suffisante de façon à obtenir 30 g de sphères hydratées.

On ajoute 30 g d'un absorbeur d'odeur cité dans l'exemple 13.

Après mélange à l'aide d'une spatule, on obtient une pâte visqueuse.

## EXEMPLE 15

A 3 g de poly β-alanine réticulée, obtenues selon l'exmple B ci-dessus, on ajoute 3 g de glycérol.
On agite à la spatule. Après un temps de gonflement de 2 heures, on ajoute 3 g de N,N-diéthyl-m-toluamide.
On obtient, après agitation, un produit semi-poudreux.

## EXEMPLE 16

A 3 g de sphères de poly β-alanine réticulée, obtenues selon FR-A-2.624.866, on ajoute 9 g d'une solution à 25 % de méthyl sulfate de [oxo-2 benzylidène-3 méthyl]-4 phényl triméthyl ammonium. On agite le milieu de façon à obtenir des sphères gonflées.
On ajoute ensuite 3 g de "ESCALOL 507", et, on homogénéise le milieu à l'aide d'une spatule.
On obtient un produit semi-poudreux.

## EXEMPLE 17

A 0,25 g de poly β-alanine sèche, on ajoute une solution de 0,02 g de nicotinate de benzyle dans 0,94 g d'un mélange éthanol/eau 50/50. Après agitation à la spatule, les sphères se trouvent gonflées par le mélange éthanol/eau contenant le nicotinate de benzyle.
On évapore ensuite l'éthanol à l'évaporateur rotatif sous pression réduite. On ajoute 0,30 g d'huile de silicone commercialisée par la société DOW CORNING sous la référence DC200 (1000 cs) silicone fluid for cosmetic use. Après homogénéisation à la spatule, on obtient un produit semi-poudreux.

## EXEMPLES DE COMPOSITIONS COSMETIQUES SOUS FORME DE GEL

## EXEMPLE 18

On prépare selon l'invention un gel homogène ayant la composition suivante :

```
Sphères imprégnées selon l'exemple 10....................  10   %
Carbopol 940...........................................  0,4 %
Triéthanolamine q.s.p......pH = 6
Eau q.s.p..............................................  100   %
```

## EXEMPLE 19

On prépare selon l'invention un gel homogène ayant le composition suivante :

```
Sphères imprégnées selon l'exemple 11....................  15   %
Carbopol 940...........................................  0,4 %
Triéthanolamine q.s.p.......pH = 6
Eau q.s.p..............................................  100   %
```

Toutes les sphères de poly β-alanine réticulée imprégnées d'une substance lipophile selon les exemples 1 à 17 peuvent être formulées sous forme d'un gel selon les exemples 18 et 19.

## EXEMPLES DE COMPOSITIONS SOUS FORME DE POUDRE

## EXEMPLE 20

On prépare selon l'invention une poudre ayant la composition suivante :

7

```
Sphères imprégnées selon l'exemple 13....................    5   %
Talc...................................................   93,9 %
Parfum.................................................    1   %
Parahydroxybenzoate de propyle.........................    0,1 %
```

EXEMPLE 21

On prépare selon l'invention une poudre ayant la composition suivante :

```
Sphères imprégnées selon l'exemple 13 ..................    5   %
Talc...................................................   90,8 %
Carbonate de magnésium.................................    3   %
Parfum.................................................    1   %
Parahydroxybenzoate de propyle.........................    0,2 %
```

EXEMPLE 22

On prépare selon l'invention un déodorant aérosol ayant la composition suivante :

```
Sphères imprégnées selon l'exemple 13 ..................    9   %
Bentone................................................    0,9 %
Myristate d'isopropyle.................................    9   %
Silicone volatil 7207 vendu par la Société UNION CARBIDE  11,1 %
Propulseur : butane à 2,8 bars.........................   70   %
```

**Revendications**

1.  Composition cosmétique ou pharmaceutique, sous forme d'un gel ou d'une poudre, contenant en dispersion des sphères de poly β-alanine réticulée imprégnées d'une substance lipophile.

2.  Composition selon la revendication 1, caractérisée par le fait que la substance lipophile est une huile ou un beurre choisie parmi l'huile de paraffine, l'huile d'arachide, l'huile de palme, une huile de silicone, ou du beurre de karité.

3.  Composition cosmétique selon la revendication 1, caractérisée par le fait que la substance lipophile est une huile essentielle choisie parmi les extraits de Rocou ou les extraits de parfum.

4.  Composition cosmétique selon la revendication 1, caractérisée par le fait que la substance lipophile est une substance active choisie parmi l'acétate d'α-tocophérol, le nicotinate d'α-tocophérol, le β-carotène, le N,N-diéthyltoluamide, un filtre solaire.

5.  Composition selon la revendication 4, caractérisée par le fait que la substance active qui imprégné les sphères est en solution dans une huile.

6.  Composition selon l'une quelconque des revendications 1 à 5 précédentes, caractérisée par le fait que les sphères de poly β-alanine réticulée imprégnées d'une substance lipophile sont présentes à une concentration comprise entre 0,1 et 60 % en poids par rapport au poids total de la composition.

7. Composition selon l'une quelconque des revendications précédentes, sous forme d'un gel, caractérisée par le fait que ledit gel est obtenu à l'aide d'un agent gélifiant présent à une concentration comprise entre 0,1 et 5 % en poids par rapport au poids total de la composition.

8. Composition selon l'une quelconque des revendications 1 à 6, sous forme d'une poudre, caractérisée par le fait que ladite poudre contient de 5 à 95 % en poids d'au moins une charge minérale ou organique.

9. Procédé de préparation des sphères de poly β-alanine réticulée imprégnées d'une substance lipophile, caractérisé par le fait que l'on soumet les sphères de poly β-alanine réticulée à une étape de gonflement à l'aide d'eau, d'un alcool polyhydroxylé ou d'un de leurs mélanges en une proportion comprise entre 1 et 10 fois la masse des sphères, et que l'on met ensuite en contact lesdites sphères en présence d'une substance lipophile de préférence sous vive agitation, et qu'enfin on isole les sphères imprégnées de la substance lipophile.

10. Procédé selon la revendication 9, caractérisé par le fait que le gonflement des sphères de poly β-alanine réticulée est réalisé à l'aide d'eau ou d'un mélange d'eau et d'un alcool polyhydroxylé choisi parmi le glycérol ou le propanediol-1,2.

11. Procédé selon la revendication 10, caractérisé par le fait que l'eau ou le mélange d'eau et d'un alcool polyhydroxylé contient au moins une substance active non-lipophile.

12. Procédé selon la revendication 9, caractérisé par le fait que le gonflement des sphères de poly β-alanine réticulée est réalisé à l'aide d'un mélange d'eau et d'un solvant miscible à l'eau choisi parmi l'éthanol, l'acétone et le tétrahydrofuranne.

13. Procédé selon la revendication 12, caractérisé par le fait que le mélange d'eau et d'un solvant miscible à l'eau contient une substance active soluble dans ce dit mélange.

14. Procédé selon l'une quelconque des revendications 9 à 13, caractérisé par le fait que l'imprégnation à l'aide de la substance lipophile est réalisée à une température comprise entre la température ambiante et une température inférieure à 100°C lorsque le gonflement a été réalisé avec de l'eau ou un mélange d'eau et d'un solvant de point d'ébullition inférieure à 100°C ou à une température supérieure à 100°C lorsque le gonflement a été réalisé avec un alcool polyhydroxylé seul ou en mélange avec de l'eau.

15. Procédé selon l'une quelconque des revendications 9 à 14, caractérisée par le fait que la substance lipophile contient au moins une substance active cosmétique ou pharmaceutique liposoluble.

## Patentansprüche

1. Kosmetische oder pharmazeutische Zusammensetzung in Form eines Gels oder eines Pulvers, enthaltend eine Dispersion von vernetzten Poly-β-alanin-Kugeln, die mit einer lipophilen Substanz imprägniert sind.

2. Zusammensetzung nach Anspruch 1, dadurch gekennzeichnet, daß die lipophile Substanz ein Öl oder eine Butter ausgewählt aus Paraffinöl, Erdnußöl, Palmöl, einem Silikonöl oder Karitébutter ist.

3. Kosmetische Zusammensetzung nach Anspruch 1, dadurch gekennzeichnet, daß die lipophile Substanz ein etherisches Öl ausgewählt aus Orleonextrakten oder Riechstoffextrakten ist.

4. Kosmetische Zusammensetzung nach Anspruch 1, dadurch gekennzeichnet, daß die lipophile Substanz ein Wirkstoff ausgewählt aus $\alpha$-Tocopherolacetat, $\alpha$-Tocopherolnicotinat, $\beta$-Carotin, N,N-Diethyltoluamid und einem Sonnenfilter ist.

5. Zusammensetzung nach Anspruch 4, dadurch gekennzeichnet, daß der Wirkstoff, der die Kugeln imprägniert, eine Lösung in einem Öl ist.

6. Zusammensetzung nach einem der vorhergehenden Ansprüche 1 bis 5, dadurch gekennzeichnet, daß die mit einer lipophilen Substanz imprägnierten, vernetzten Poly-β-alanin-Kugeln in einer Konzentration zwischen 0,1 und 60 Gew.-% einschließlich, bezogen auf das Gesamtgewicht der Zusammensetzung, vorliegen.

7. Zusammensetzung nach einem der vorhergehenden Ansprüche in Form eines Gels, dadurch gekennzeichnet, daß das Gel mit Hilfe eines Geliermittels erhalten wird, das in einer Konzentration zwischen 0,1 und 5 Gew.-% einschließlich, bezogen auf das Gesamtgewicht der Zusammensetzung, vorhanden ist.

8. Zusammensetzung nach einem der Ansprüche 1 bis 6 in Form eines Pulvers, dadurch gekennzeichnet, daß das Pulver 5 bis 95 Gew.-% mindestens eines mineralischen oder organischen Füllstoffs enthält.

9. Verfahren zur Herstellung der mit einer lipophilen Substanz imprägnierten, vernetzten Poly-β-alanin-Kugeln, dadurch gekennzeichnet, daß man die vernetzten Poly-β-alanin-Kugeln in einem ersten Schritt einer Quellung mit Wasser, einem mehrwertigen Alkohol oder Mischungen davon in einer Menge, die zwischen dem 1- und 10fachen der Masse der Kugeln liegt, unterwirft und anschließend die Kugeln mit der lipophilen Substanz vorzugsweise unter starkem Rühren in Kontakt bringt und abschließend die mit der lipophilen Substanz imprägnierten Kugeln isoliert.

10. Verfahren nach Anspruch 9, dadurch gekennzeichnet, daß man die Quellung der vernetzten Poly-β-alanin-Kugeln mittels Wasser oder einer Mischung aus Wasser und einem mehrwertigen Alkohol, ausgewählt aus Glycerin oder 1,2-Propandiol, durchführt.

11. Verfahren nach Anspruch 10, dadurch gekennzeichnet, daß das Wasser oder die Mischung aus Wasser und einem mehrwertigen Alkohol mindestens einen nicht-lipophilen Wirkstoff enthält.

12. Verfahren nach Anspruch 9, dadurch gekennzeichnet, daß man die Quellung der vernetzten Poly-β-alanin-Kugeln mittels einer Mischung aus Wasser und einem mit Wasser mischbaren Lösungsmittel, ausgewählt aus Ethanol, Aceton und Tetrahydrofuran, durchführt.

13. Verfahren nach Anspruch 12, dadurch gekennzeichnet, daß die Mischung aus Wasser und einem mit Wasser mischbaren Lösungsmittel einen Wirkstoff enthält, der in dieser Mischung löslich ist.

14. Verfahren nach einem der vorhergehenden Ansprüche 9 bis 13, dadurch gekennzeichnet, daß man die Imprägnierung mit der lipophilen Substanz bei einer Temperatur zwischen Raumtemperatur und einer Temperatur unter 100°C einschließlich durchführt, wenn die Quellung mit Wasser oder einer Mischung aus Wasser und einem Lösungsmittel mit einem Siedepunkt unter 100°C durchgeführt wurde, oder bei einer Temperatur über 100°C durchführt, wenn die Quellung mit einem mehrwertigen Alkohol allein oder in Mischung mit Wasser durchgeführt wurde.

15. Verfahren nach einem der Ansprüche 9 bis 14, dadurch gekennzeichnet, daß die lipophile Substanz mindestens einen fettlöslichen kosmetischen der pharmazeutischen Wirkstoff enthält.

## Claims

1. Cosmetic or pharmaceutical composition in the form of a gel or a powder containing in dispersion crosslinked poly(β-alanine) spheres impregnated with a lipophilic substance.

2. Composition according to Claim 1, characterised in that the lipophilic substance is an oil or a butter chosen from paraffin oil, groundnut oil, palm oil, a silicone oil or karite butter.

3. Cosmetic composition according to claim 1, characterised in that the lipophilic substance is an essential oil chosen from annatto extracts or perfume extracts.

4. Cosmetic composition according to Claim 1, characterised in that the lipophilic substance is an active substance chosen from α-tocopherol acetate, α-tocopherol nicotinate, β-carotene, N,N-diethyltoluamide, a sunscreen agent.

5. Composition according to Claim 4, characterised in that the active substance which impregnates the spheres is in solution in an oil.

6. Composition according to any one of Claims 1 to 5 above, characterised in that the crosslinked poly(β-alanine) spheres, impregnated with a lipophilic substance, are present at a concentration of between 0.1 and 60 % by weight relative to the total weight of the composition.

7. Composition according to any one of the above claims, in the form of a gel, characterised in that the said gel is obtained by means of a gelling agent present at a concentration of between 0.1 and 5 % by weight relative to the total weight of the composition.

8. Composition according to any one of Claims 1 to 6, in the form of a powder, characterised in that the said powder contains 5 to 95 % by weight of at least one inorganic or organic filler.

9. Process for preparing crosslinked poly(β-alanine spheres impregnated with a lipophilic substance, characterised in that the crosslinked poly(β-alanine) spheres are subjected to a step of swelling by means of water, a polyhydroxylated alcohol or one of their mixtures in a proportion of between 1 and 10 times the mass of the spheres, and in that the said spheres are then brought into contact in the presence of a lipophilic substance, preferably with vigourous stirring, and in that finally the spheres, impregnated with the lipophilic substance, are isolated.

10. Process according to Claim 9, characterised in that the swelling of the crosslinked poly(β-alanine) spheres is carried out by means of water or a mixture of water and a polyhydroxylated alcohol chosen from glycerol or 1,2-propanediol.

11. Process according to Claim 10, characterised in that the water or the mixture of water and a polyhydroxylated alcohol contains at least one non-lipophilic active substance.

12. Process according to Claim 9, characterised in that the swelling of the crosslinked poly(β-alanine) spheres is carried out by means of a mixture of water and a water-miscible solvent chosen from ethanol, acetone and tetrahydrofuran.

13. Process according to Claim 12, characterised in that the mixture of water and a water-miscible solvent contains an active substance soluble in this said mixture.

14. Process according to any one of Claims 9 to 13, characterised in that the impregnation by means of the lipophilic substance is carried out at a temperature of between room temperature and a temperature below 100°C when the swelling was carried out using water or a mixture of water and a solvent with a boiling point below 100°C or at a temperature above 100°C when the swelling was carried out using a polyhydroxylated alcohol alone or mixed with water.

15. Process according to any one of Claims 9 to 14, characterised in that the lipophilic substance contains at least one fat-soluble cosmetically or pharmaceutically active substance.